(19) Europäisches Patentamt
European Patent Office
Office européen des brevets

(11) Numéro de publication: **0 351 283 A1**

(12) **DEMANDE DE BREVET EUROPEEN**

(21) Numéro de dépôt: **89401899.3**

(22) Date de dépôt: **03.07.89**

(51) Int. Cl.[5]: **C 07 D 401/06**
A 61 K 31/445, A 61 K 31/55

(30) Priorité: **12.07.88 FR 8809450**
**12.07.88 FR 8809451**

(43) Date de publication de la demande:
**17.01.90 Bulletin 90/03**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **George, Pascal**
**19, rue des Quatre Vents**
**F-78730 St Arnoult en Yvelines (FR)**

**Sevrin, Mireille**
**73, rue Raymond Losserand**
**F-75014 Paris (FR)**

**Mangane, Michel**
**44, Avenue Marcel Cachin**
**F-92320 Chatillon S/Bagneux (FR)**

**Merly, Jean-Pierre**
**11, avenue Jules Guesde**
**F-92330 Sceaux (FR)**

**Bigg, Dennis**
**30, Avenue de Lavaur**
**F-81100 Castres (FR)**

(74) Mandataire: **Ludwig, Jacques et al**
**SYNTHELABO Service Brevets 58, rue de la Glacière**
**F-75013 Paris (FR)**

Revendications pour les Etats contractants suivants: ES + GR.

(54) **Dérivés de [(pipéridinyl-4) méthyl]-2 dihydro-2,3 1H-isoindole et tétrahydro-2,3,4,5 1H-benzazépines, leur préparation et leur application en thérapeutique.**

(57) Composés de formule générale (I)

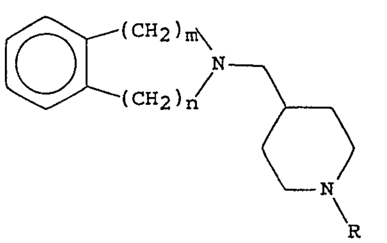

(I)

dans laquelle
soit chacun des symboles m et n représente le nombre 1,
soit chacun des symboles m et n représente le nombre 2,
soit m représente le nombre 3 et n représente le nombre 1, et R représente soit un atome d'hydrogène, soit un groupe de formule générale -Z-R′ dans laquelle Z représente un groupe -CO- ou un groupe $-CH_2-$ et R′ représente un groupe phényle portant éventuellement de un à trois substituants choisis parmi les atomes d'halogènes et les groupes $(C_1-C_3)$alkyle et $(C_1-C_3)$alcoxy.
Application en thérapeutique.

EP 0 351 283 A1

## Description

### DERIVES DE [(PIPERIDINYL-4)METHYL] DIHYDRO-2,3 1H-ISOINDOLE ET TETRAHYDRO-2,3,4,5 1H-BENZAZEPINES, LEUR PREPARATION ET LEUR APPLICATION EN THERAPEUTIQUE

La présente demande de brevet a pour objet des dérivés de [(pipéridinyl-4)méthyl] dihydro-2,3 1*H*-isoindole et tétrahydro-2,3,4,5 1*H*benzazépines, leur préparation et leur application en thérapeutique.

Les composés de l'invention répondent à la formule générale

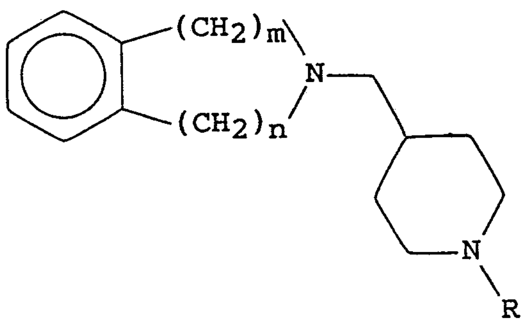

(I)

dans laquelle
soit chacun des symboles m et n représente le nombre 1,
soit chacun des symboles m et n représente le nombre 2,
soit m représente le nombre 3 et n représente le nombre 1, et
R représente soit un atome d'hydrogène, soit un groupe de formule générale -Z-R′ dans laquelle Z représente un groupe -CO- ou un groupe $-CH_2-$ et R′ représente un groupe phényle portant éventuellement de un à trois substituants choisis parmi les atomes d'halogènes et les groupes $(C_1-C_3)$alkyle linéaires ou ramifiés et $(C_1-C_3)$alcoxy linéaires ou ramifiés.

Ils peuvent se présenter à l'état de bases libres ou de sels d'addition à des acides.

Conformément à l'invention on peut préparer les composés de formule générale (I) par un procédé illustré par le schéma 1 donné ci-après.

On prépare d'abord un composé de formule générale (I) où Z représente un groupe -CO- en faisant réagir un composé de formule générale (II) (dans laquelle m et n sont tels que définis ci-dessus) avec un tosylate de formule générale (III) (dans laquelle Tos représente un groupe tosyle et R′ est tel que défini ci-dessus), soit en l'absence, soit en présence d'un solvant inerte tel que le diméthylformamide, le toluène

Schéma 1

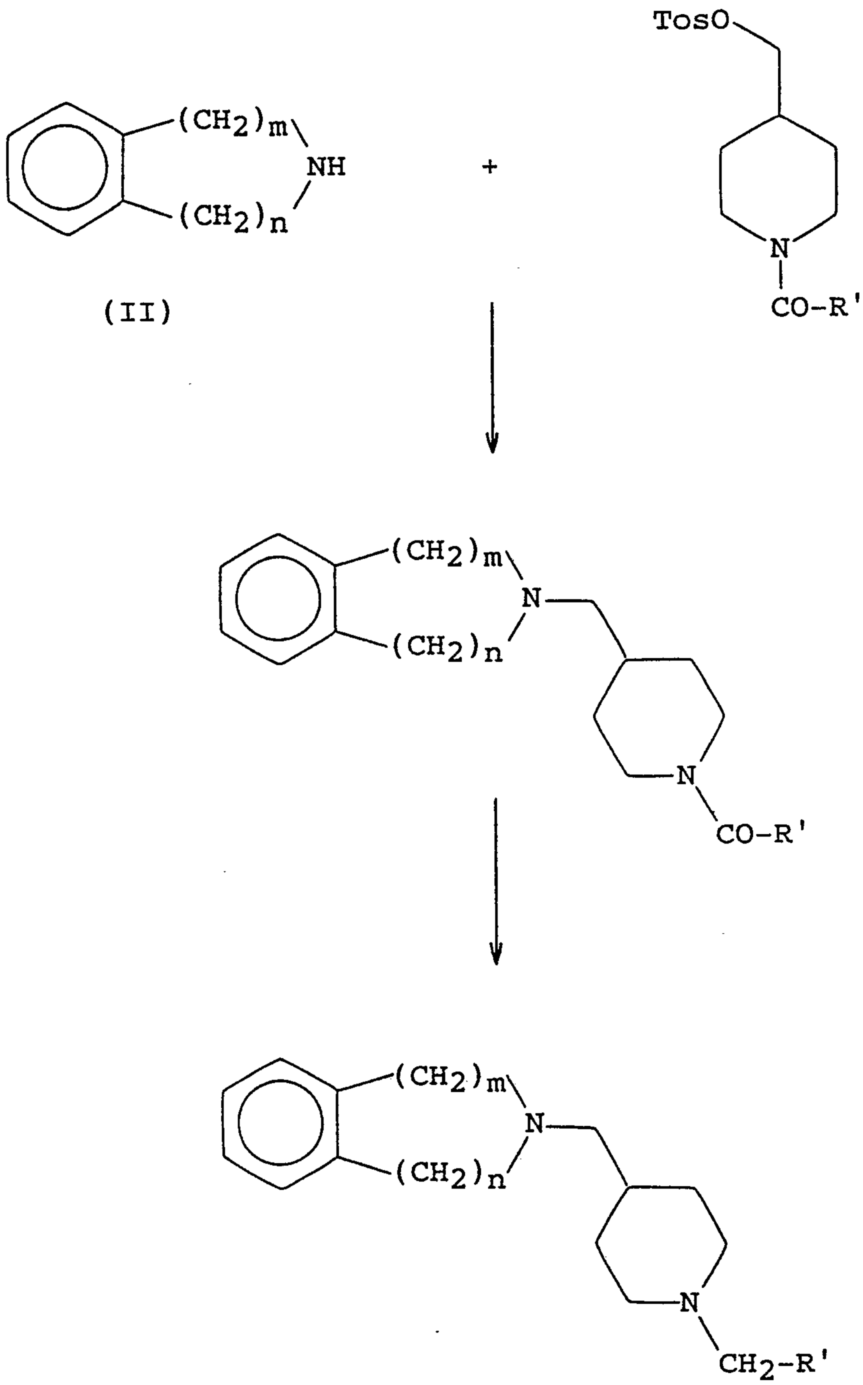

(II) (III) (Ia) (Ib)

ou le xylène, à une température de 20 à 150° C, et éventuellement en présence d'une base organique, telle qu'une amine tertiaire, ou minérale, telle qu'un carbonate ou hydrogénocarbonate alcalin.
On obtient ainsi un composé de formule générale (Ia) qui correspond à la formule générale (I) lorsque Z représente un groupe -CO-.

Si l'on désire préparer un composé de formule générale (I) où Z représente un groupe $-CH_2-$, on réduit ensuite le composé de formule générale (Ia) au moyen d'un hydrure simple ou complexe de bore ou d'aluminium, par exemple l'hydrure de lithium et d'aluminium, l'hydrure d'aluminium, le complexe diborane/tétrahydrofuranne ou le complexe diborane/sulfure de méthyle, ou tout moyen équivalent, dans un solvant éthéré tel que l'éther diéthylique, le tétrahydrofuranne ou le dioxanne, à une température de 20 à 100° C.
On obtient ainsi un composé de formule générale (Ib) qui correspond à la formule générale (I) lorsque Z représente un groupe $-CH_2-$.

Enfin, les composés de formule générale (I) où R représente un atome d'hydrogène peuvent être obtenus par débenzylation des composés de formule générale (I) où R représente un groupe benzyle, par exemple au moyen d'une hydrogénolyse par l'hydrogène gazeux, sous une pression de 0,1 à 0,5 MPa, en présence d'un catalyseur métallique, éventuellement sur support inerte ou basique, par exemple le palladium sur charbon, sulfate de baryum ou carbonate de calcium, dans un solvant alcoolique, par exemple le méthanol ou l'éthanol, à une température de 20 à 80°C.

Le dihydro-2,3 1*H*-isoindole de formule (II) (m = n = 1) peut être obtenu par un procédé tel que celui décrit dans Organic Syntheses, Collective Vol. V, 406-408 et 1064-1066

La tétrahydro-2,3,4,5 1*H*-benzazépine-3 de formule générale (II) (m = n = 2) peut être obtenue par un procédé tel que celui décrit dans Helvetica Chim. Acta, 18, 1388, (1935).

La tétrahydro-2,3,4,5 1*H*-benzazépine-2 de formule générale (II) (m = 3 et n = 1) peut être obtenue par un procédé tel que celui décrit dans J.C.S., Perkin I, 782 (1973).

Les tosylates de formule générale (III) peuvent être préparés selon une méthode illustrée par le schéma 2 qui suit. On fait réagir le pipéridine-4-méthanol de formule (IV) avec un chlorure d'acide de formule générale R'COCl (dans laquelle R' est tel que défini ci-dessus), dans un solvant inerte tel qu'un solvant chloré, à une température de 20 à 80°C. On obtient ainsi un ester-amide de formule générale (V), qu'on saponifie , par exemple au moyen d'hydroxyde de sodium ou de potassium, dans un solvant alcoolique aliphatique inférieur, de préférence l'éthanol, pour obtenir l'alcool de formule générale (VI), dont on prépare finalement le tosylate au moyen de chlorure de tosyle, dans un milieu basique tel que la pyridine.

Le pipéridine-4-méthanol de formule (IV) peut être obtenu par exemple par réduction de pipéridine-4-carboxylate d'éthyle au moyen d'hydrure de lithium et aluminium, ou encore par une telle réduction de benzyl-1 pipéridine-4-carboxylate d'éthyle suivie d'une hydrogénolyse catalytique sous pression.

Enfin, une autre variante de procédé, dont le détail est illustré par l'exemple 3 ci-après, permet de préparer les composés de l'invention à partir de di(bromoalkyl)-1,2 benzènes et de phénylméthyl-1 pipéridine-4-carboxamides ou -4-méthanamines convenablement choisis, avec formation du cycle azoté du dihydro-2,3 1*H*-indole ou des tétrahydro2,3,4,5 1*H*-benzazépines.

Les exemples suivants illustrent en détail la préparation de quelques composés selon l'invention. Les microanalyses élémentaires et les spectres IR et RMN confirment les structures des produits obtenus.

Les numéros indiqués entre parenthèses dans les titres des exemples correspondent à ceux du tableau donné plus loin.

Schéma 2

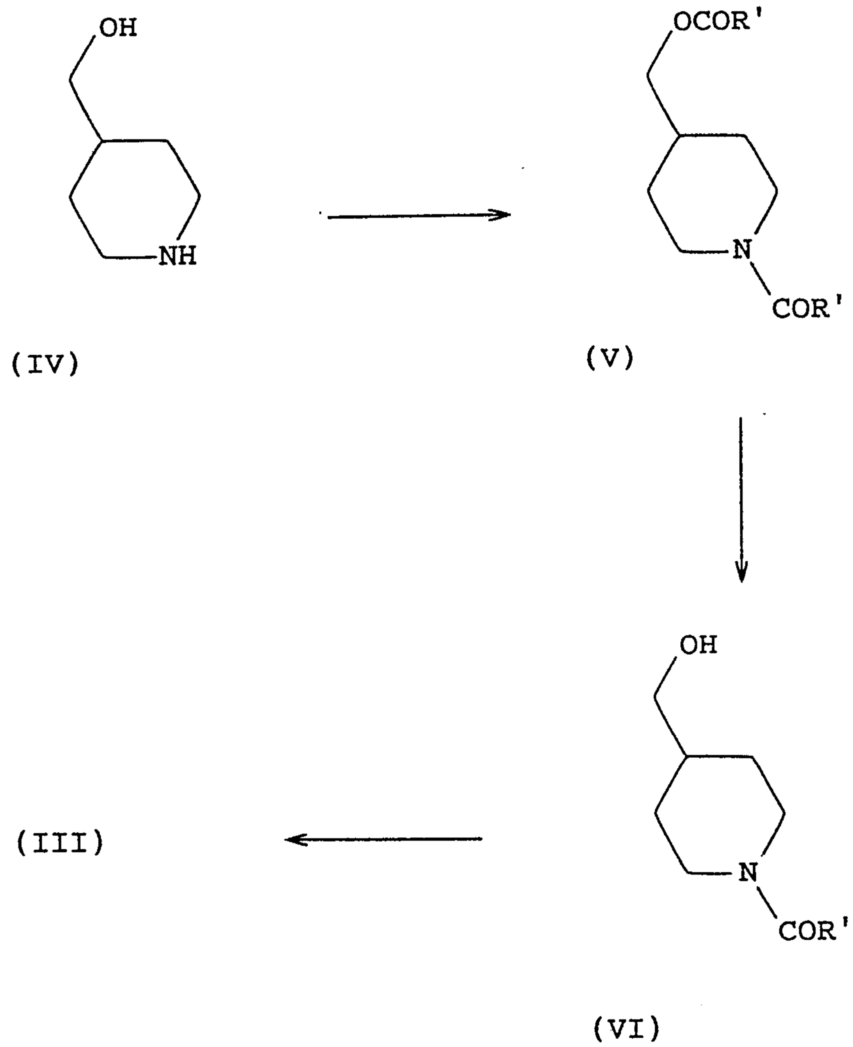

(IV) (V) (VI) (III)

Exemple 1 (Composé N°6)

[[[(Méthyl-3 phényl)carbonyl]-1 pipéridinyl-4]méthyl]-2 dihydro-2,3 1*H*-isoindole, chlorhydrate.

1.1 Pipéridine-4-méthanol.

Dans un ballon tricol de 4 l muni d'un système d'agitation mécanique et d'un réfrigérant on introduit 28,5 g (0,75 mole) d'hydrure de lithium et aluminium et 1,2 l de tétrahydrofuranne. On ajoute à la suspension obtenue 117,9 g (0,75 mole) de pipéridine-4-carboxylate d'éthyle en solution dans 1,2 l de tétrahydrofuranne, et on agite le mélange pendant 6h à 20°C. On le refroidit à 0°C, puis on l'hydrolyse en ajoutant successivement 22 ml d'eau, 22 ml d'hydroxyde de sodium 1N et 46 ml d'eau. On agite le mélange 30mn à 20°C, on le filtre, on lave le précipité au tétrahydrofuranne puis à l'éther. On évapore les solvants sous pression réduite, et on obtient 84,4 g d'une huile qu'on utilise telle quelle dans l'étape suivante.

1.2. Méthyl-3 benzoate de [[(méthyl-3 phényl)carbonyl]-1 pipéridinyl-4]méthyle.

Dans un ballon tricol de 3 l on introduit, sous atmosphère d'argon, 42,25 g (0,367 mole) de pipéridine-4-méthanol, 430 ml de dichloro-1,2 éthane, et on ajoute 82 g (0,81 mole) de triéthylamine puis 125,2 g (0,81 mole) de chlorure de méthyl-3 benzoyle. On chauffe le mélange au reflux pendant 4h30, on ajoute

encore 8,2 g (0,08 mole) de triéthylamine et 12,5 g (0,08 mole) de chlorure de méthyl-3 benzoyle, et on chauffe le mélange pendant encore 3h.
On le filtre, on lave les sels au dichloro-1,2 éthane, on évapore le filtrat sous pression réduite, on dissout le résidu dans de l'acétate d'éthyle, on lave la solution avec une solution aqueuse saturée de chlorure de sodium, on évapore le solvant sous pression réduite, et on recristallise le résidu dans un mélange 1/1 d'alcool isopropylique/acétate d'éthyle. On obtient 80 g d'un solide blanc. Point de fusion : 80-83° C.

1.3. [Méthyl-3 phényl)carbonyl]-1 pipéridine-4-méthanol.

A une solution de 80 g (0,23 mole) de méthyl-3 benzoate de [[(méthyl-3 phényl)carbonyl]-1 pipéridinyl-4]méthyle dans 400 ml d'éthanol, on ajoute une solution de 12,76 g (0,23 mole) d'hydroxyde de potassium dans 75 ml d'éthanol et 75 ml d'eau. On agite le mélange à 20° C pendant 3h, on évapore l'éthanol sous pression réduite et on extrait la phase aqueuse à l'acétate d'éthyle. On lave la phase organique avec de l'eau puis avec une solution aqueuse saturée de chlorure de sodium, et on la sèche sur sulfate de magnésium. On évapore le solvant sous pression réduite et on obtient 53 g d'alcool qu'on utilise tel quel dans l'étape suivante.

1.4. (Méthyl-4 phényl)sulfonate de [[(méthyl-3 phényl)carbonyl]-1 pipéridinyl-4]méthyle.

A une solution de 52 g (0,22 mole) de [(méthyl-3 phényl)carbonyl]-1 pipéridine-4-méthanol dans 100 ml de pyridine on ajoute 53,3 g (0,28 mole) de chlorure de méthyl-4 phénylsulfonyle dans 60 ml de pyridine. On agite le mélange à 20° C pendant 4h, puis on le verse dans de la glace. On extrait la phase au dichlorométhane, on lave la phase organique avec une solution aqueuse 10N d'acide chlorhydrique et on la sèche sur sulfate de magnésium. On évapore les solvants sous pression réduite et on obtient 70 g de solide blanc. Point de fusion : 68-70° C.

1.5. [[[(Méthyl-3 phényl)carbonyl]-1 pipéridinyl-4]méthyl]-2 dihydro-2,3 1*H*-isoindole, chlorhydrate.

Dans un ballon de 250 ml, placé sous atmosphère d'argon, on introduit 3,6 g (0,03 mole) de dihydro-2,3 1*H*-isoindole et 12,8 g (0,033 mole) de (méthyl-4 phényl)sulfonate de [[(méthyl-3 phényl)carbonyl]-1 pipéridinyl-4]méthyle et on chauffe le mélange à 150° C pendant 3h30. On obtient une huile épaisse de couleur marron qu'on dilue avec du dichlorométhane, on ajoute de l'ammoniaque concentrée, on sépare la phase organique, on la lave avec de l'eau, on la sèche sur sulfate de magnésium, on évapore les solvants sous pression réduite et on purifie le résidu par chromatographie sur colonne de silice. On isole 7,5 g de base. Point de fusion : 125-127° C.

On en prépare le chlorhydrate au moyen de propanol-2 chlorhydrique et on le recristallise dans un mélange d'acétate d'éthyle/propanol-2. Point de fusion : 217,5-220° C.

Exemple 2 (Composé N° 7)

[[[(Méthyl-3 phényl)méthyl]-1 pipéridinyl-4]méthyl]-2 dihydro-2,3 1*H*-isoindole, difumarate.

Dans un ballon tricol de 250 ml, placé sous atmosphère d'argon, on introduit 0,54 g (14,4 mmoles) d'hydrure de lithium et aluminium dans 20 ml de tétrahydrofuranne, on ajoute 3,15 g (9,42 mmoles) de [[[(méthyl-3 phényl)carbonyl]-1 pipéridinyl-4]méthyl]-2 dihydro-2,3 1*H*-isoindole en solution dans 75 ml de tétrahydrofuranne refroidis à 0° C, on agite le mélange à froid pendant 5mn puis on le chauffe au reflux pendant 1h. On obtient une solution limpide orange à laquelle on ajoute 7 ml d'hydroxyde de sodium à 6,5%, en la refroidissant. On obtient une suspension orange foncé, on la filtre, on sèche le filtrat sur sulfate de magnésium, on évapore le solvant sous pression réduite et on purifie le résidu par chromatographie sur colonne de silice en éluant avec un mélange 96/4 de dichlorométhane/méthanol. On isole 2,35 g d'une huile brune qui cristallise. Point de fusion : 93,5-95° C.
On dissout cette base (7,33 mmoles) dans 150 ml d'éthanol, on ajoute une solution de 1,7 g (14,7 mmoles) d'acide fumarique dans 100 ml d'éthanol, on concentre le mélange jusqu'à moitié de son volume initial, on sépare par filtration le solide blanc qui cristallise et on le recristallise dans un mélange 1/1 d'acétate d'éthyle/éthanol. On obtient finalement 2,8 g de difumarate pur. Point de fusion : 198-200,5° C.

Exemple 3 (Composé N° 3)

[[(Phénylméthyl)-1 pipéridinyl-4]méthyl]-2 dihydro-2,3 1*H*-isoindole, dichlorhydrate.

A. Première variante.

3.A.1. (Phénylméthyl)-1 pipéridine-4-carboxamide.

Sous atmosphère d'argon on introduit 123,0 g (0,96 mole) de pipéridine-4-carboxamide et 90,8 g (1,08 mole) de bicarbonate de sodium dans 2 l de toluène sec. On ajoute 180,6 g, soit 125,6 ml (1,056 mole) de bromométhylbenzène et on chauffe le mélange au reflux pendant 5h.
On filtre le mélange à chaud. Pendant le refroidissement du filtrat il se forme un précipité blanc. On l'isole, on le sèche, et on obtient 113,4 g de produit sec. Point de fusion : 160-162° C.

3.A.2. [[(Phénylméthyl)-1 pipéridinyl-4]carbonyl]-2 dihydro-2,3 1*H*-isoindole, chlorhydrate.

Sous atmosphère d'argon on introduit dans un ballon 2,3 g d'hydrure de sodium à 50 % dans l'huile (48,1 mmoles), préalablement lavé à l'éther de pétrole, puis 100 ml de diméthylformamide, puis une solution de 5 g (22,9 mmoles) de (phénylméthyl)-1 pipéridine-4-carboxamide dans 45 ml de diméthylformamide, à température ambiante et dans un délai de 20 mn. On agite la suspension pendant 1h à température ambiante puis 1h à 60°C.

On ajoute ensuite une solution de 6,05 g (22,9 mmoles) de di(bromométhyl)-1,2 benzène dans 40 ml de diméthylformamide, de façon à maintenir la température entre 60 et 70°C, et on agite le mélange pendant 3h à température ambiante.

On le verse sur un mélange d'eau et de glace, on le traite avec de l'acétate d'éthyle, on sépare la phase organique, on la lave à l'eau, on la sèche et on évapore le solvant. On obtient un solide orange qu'on recristallise dans le cyclohexane, lave à l'éther, et recristallise encore une fois dans le cyclohexane. On obtient 1,1 g de solide blanc.

On l'introduit dans un mélange de 50 ml d'alcool isopropylique et de 34,3 ml d'alcool isopropylique chlorhydrique 0,1N, on chauffe au reflux et on laisse refroidir la solution. On obtient 1,2 g de chlorhydrate. Point de fusion : 243-246°C (décomposition).

3.A.3. [[(Phénylméthyl)-1 pipéridinyl-4]méthyl]-2 dihydro-2,3 1*H*-isoindole, dichlorhydrate.

On ajoute lentement une solution de 10,0 g (31,2 mmoles) de [[(phénylméthyl)-1 pipéridinyl-4]carbonyl]-2 dihydro-2,3 1*H*-isoindole dans 20 mld'éther sec, à froid, à 1,85 g (48,4 mmoles) d'hydrure de lithium et d'aluminium dans 300 ml d'éther sec. On agite le mélange pendant 30 mn à température ambiante, puis pendant 7h au reflux. Puis on l'hydrolyse avec 8 ml d'eau, on le filtre en rinçant le solide avec de l'éther, on sèche le filtrat et on le concentre. On obtient une huile jaune qui cristallise rapidement. On la reprend avec de l'éthanol et on fait passer dans la solution un courant d'acide chlorhydrique gazeux. On obtient un solide de couleur crème qu'on recristallise deux fois dans un mélange 2/1 d'éthanol/méthanol. On obtient 7,7 g de dichlorhydrate. Point de fusion : 291-293°C.

B. Deuxième variante.

3.B.1. (Phénylméthyl)-1 pipéridine-4-méthanamine, chlorhydrate.

Sous atmosphère d'argon on introduit 50,0 g (229 mmoles) de (phénylméthyl)-1 pipéridine-4-carboxamide et 13,0 g (343 mmoles) d'hydrure de lithium et d'aluminium dans 2,5 l d'éther sec, et on chauffe le mélange au reflux pendant 8h. On ajoute 50 ml d'eau, on filtre le mélange en rinçant le solide à l'éther, on sépare la phase organique, on la sèche et on évapore le solvant. On obtient 40,7 g d'une huile jaune qu'on dissout dans 1,99 1 d'alcool isopropylique chlorhydrique 0,1N. On concentre la solution aux 3/4, on laisse refroidir, on filtre le solide blanc qui a précipité et on le recristallise dans l'alcool isopropylique. On obtient 33,7 g de chlorhydrate. Point de fusion : 188-190°C.

3.B.2. [[(Phénylméthyl)-1 pipéridinyl-4]méthyl]-2 dihydro-2,3 1*H*-isoindole, dichlorhydrate.

Sous atmosphère d'argon on introduit 1,97 g (7,48 mmoles) de di(bromométhyl)-1,2 benzène et 5,2 g (3,74 mmoles) de carbonate de potassium dans 40 ml de diméthylformamide. On refroidit le mélange par un bain de glace et on ajoute lentement 1,8 g de chlorhydrate de (phénylméthyl)-1 pipéridine-4-méthanamine.

On agite le mélange pendant 4h à température ambiante puis on le verse sur un mélange d'eau et de glace. On extrait le solide avec de l'acétate d'éthyle, on sépare la phase organique, on la lave à l'eau, on la sèche et on évapore le solvant. On obtient 1,8 g d'une huile jaune qui cristallise, et qu'on dissout à chaud dans 59 ml d'alcool isopropylique chlorhydrique 0,1N. On laisse refroidir, on filtre le solide blanc qui a précipité et on isole 0,8 g de dichlorhydrate. Point de fusion: 291-291,5°C.

<u>Exemple 4</u> (Composé N°1)

[(Pipéridinyl-4)méthyl]-2 dihydro-2,3 1*H*-isoindole, dichlorhydrate.

On introduit 56,6 g (149 mmoles) de dichlorhydrate de [[(phénylméthyl)-1 pipéridinyl-4]méthyl]-2 dihydro-2,3 1*H*-isoindole, 300 ml d'éthanol, 50 ml d'eau et 5 g de charbon palladié à 10% dans un appareil de Parr et on effectue une hydrogénolyse sous environ 0,41 MPa pendant 9h. On filtre le mélange obtenu, on évapore le filtrat et on reprend le résidu avec un mélange 1/1 de méthanol/éthanol en présence de charbon activé. On filtre, on évapore le filtrat et on obtient 39,6 g de dichlorhydrate blanc légèrement bleuté. Point de fusion : 298-301°C.

<u>Exemple 5</u> (Composé N°14)

[[[(Méthyl-3 phényl)carbonyl]-1 pipéridinyl-4]méthyl]-2 tétrahydro-2,3,4,5 1*H*-benzazépine-3, fumarate.

Dans un ballon placé sous argon et muni d'un système d'agitation on introduit 3 g (0,02 mole) de tétrahydro-2,3,4,5 1*H*-benzazépine-3, 7,6 g (0,02 mole) de méthyl-4 benzènesulfonate de [[(méthyl-3 phényl)carbonyl]-1 pipéridinyl-4]méthyle, 2,8 g (0,02 mole) de carbonate de potassium et 20 ml de diméthylformamide. On agite le mélange pendant 9h à 90°C, on le refroidit et on le verse dans de l'eau. On

extrait le mélange avec du dichlorométhane, on lave la phase organique avec de l'eau, on la sèche sur sulfate de magnésium et on évapore les solvants sous pression réduite. On obtient 7,5 g de résidu qu'on purifie par chromatographie sur colonne de silice en éluant avec de l'éther, ce qui fournit 2 g de base pure. On en dissout 1 g dans le minimum d'éthanol, on ajoute 0,33 g d'acide fumarique, on agite le mélange pendant 30mn à température ambiante, on évapore le solvant sous pression réduite et on recristallise le résidu dans l'alcool isopropylique. On isole finalement 0,75 g de fumarate. Point de fusion : 159-160°C.

Exemple 6 (Composé N°15)

[[[(Méthyl-3 phényl)méthyl]-1 pipéridinyl-4]méthyl]-2 tétrahydro-2,3,4,5 1*h*-benzazépine-3, difumarate.

A une suspension de 0,26 g (7 mmoles) d'hydrure de lithium et aluminium dans 10 ml de tétrahydrofuranne on ajoute, sous atmosphère d'argon, 1 g (2,8 mmoles) de [[[(méthyl-3 phényl) carbonyl]-1 pipéridinyl-4]méthyl]-2 tétrahydro-2,3,4,5 1*H*-benzazépine-3 en solution dans 50 ml de tétrahydrofuranne. On agite le mélange à 60°C pendant 2h, on l'hydrolyse en ajoutant successivement 1 ml d'eau et 2 ml de soude 1N, on le sèche sur sulfate de magnésium, on le filtre et on évapore le filtrat sous pression réduite. On obtient 0,8 g de résidu huileux qu'on reprend avec le minimum d'éthanol, on ajoute 0,55 g d'acide fumarique en solution dans l'éthanol, et on filtre les cristaux blancs qui précipitent. On isole finalement 0,5 g de difumarate. Point de fusion : 212-213°C.

Exemple 7 (Composé N°20)

[[[(Méthyl-3 phényl)carbonyl]-1 pipéridinyl-4]méthyl]-2 tétrahydro-2,3,4,5 1*H*-benzazépine-2, chlorhydrate.

Dans un ballon placé sous argon et muni d'un système d'agitation on introduit 3 g (0,02 mole) de tétrahydro-2,3,4,5 1*H*-benzazépine-2, 7,6 g (0,02 mole) de méthyl-4 benzènesulfonate de [[(méthyl-3 phényl)carbonyl]-1 pipéridinyl-4]méthyle, 2,8 g (0,02 mole) de carbonate de potassium et 20 ml de diméthylformamide. On agite le mélange pendant 9h à 90°C, on le refroidit et on le verse dans de l'eau. On extrait le mélange avec du dichlorométhane, on lave la phase organique avec de l'eau, on la sèche sur sulfate de magnésium et on évapore les solvants sous pression réduite. On obtient 6,5 g de résidu qu'on purifie par chromatographie sur colonne de silice en éluant avec de l'éther, ce qui fournit 2,5 g de base pure. On en dissout 1 g dans le minimum d'éthanol, on ajoute 28 ml de propanol-2 chlorhydrique 0,1N, on agite le mélange pendant 30mn à température ambiante, on évapore le solvant sous pression réduite et on recristallise le résidu dans l'alcool isopropylique. On isole finalement 0,6 g de chlorhydrate. Point de fusion : 192-193°C.

Exemple 8 (Composé N°21)

[[[(Méthyl-3 phényl)méthyl]-1 pipéridinyl-4]méthyl]-2 tétrahydro-2,3,4,5 1*H*-benzazépine-2, difumarate.

A une suspension de 0,314 g (8,2 mmoles) d'hydrure de lithium et aluminium dans 20 ml de tétrahydrofuranne on ajoute, sous atmosphère d'argon, 1,5 g (4,1 mmoles) de [[[(méthyl-3 phényl) carbonyl]-1 pipéridinyl-4]méthyl]-2 tétrahydro-2,3,4,5 1*H*-benzazépine-2 en solution dans 20 ml de tétrahydrofuranne. On agite le mélange à 60°C pendant 2h, on l'hydrolyse en ajoutant successivement 1 ml d'eau et 2 ml de soude 1N, on le sèche sur sulfate de magnésium, on le filtre et on évapore le filtrat sous pression réduite. On obtient 1,1 g de résidu huileux qu'on reprend avec le minimum d'éthanol, on ajoute 0,67 g d'acide fumarique en solution dans l'éthanol, et on filtre les cristaux blancs qui précipitent. On isole finalement 1,2 g de difumarate. Point de fusion : 176-177°C.

Le tableau ci-après illustre les structures chimiques et les propriétés physiques de quelques composés selon l'invention. Dans la colonne "Sel", "HCl" désigne le chlorhydrate, "diHCl" désigne le dichlorhydrate, "fum." désigne le fumarate, et "difum." désigne le difumarate. L'astérisque * désigne un sel dihydraté.

Tableau

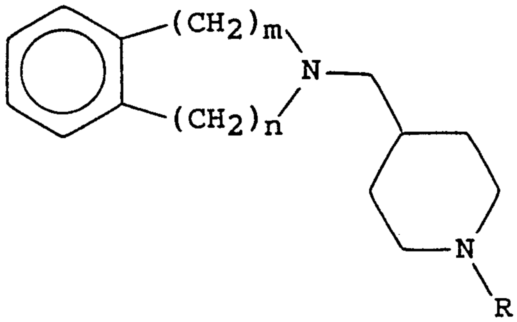

| N° | m | n | R | Sel | F(°C) |
|---|---|---|---|---|---|
| 1 | 1 | 1 | H | diHCl | 298-301 |
| 2 | 1 | 1 | $C_6H_5$-CO- | HCl | 225-227 |
| 3 | 1 | 1 | $C_6H_5$-$CH_2$- | diHCl | 291-293 |
| 4 | 1 | 1 | Cl-3-$C_6H_4$-CO- | fum. | 141-143 |
| 5 | 1 | 1 | Cl-3-$C_6H_4$-$CH_2$- | difum. | 198,5-200,5 |
| 6 | 1 | 1 | $CH_3$-3-$C_6H_4$-CO- | HCl | 217,5-220 |
| 7 | 1 | 1 | $CH_3$-3-$C_6H_4$-$CH_2$- | difum. | 198-200,5 |
| 8 | 1 | 1 | $C_2H_5O$-3-$C_6H_4$-CO- | HCl | 171-173 |
| 9 | 1 | 1 | $C_2H_5O$-3-$C_6H_4$-$CH_2$- | difum. | 206,5-208 |
| 10 | 2 | 2 | $C_6H_5$-CO- | fum. | 210-212 |
| 11 | 2 | 2 | $C_6H_5$-$CH_2$- | difum. | 220-222 |
| 12 | 2 | 2 | Cl-3-$C_6H_4$-CO- | fum. | 197-198 |
| 13 | 2 | 2 | Cl-3-$C_6H_4$-$CH_2$- | difum. | 189-190 |
| 14 | 2 | 2 | $CH_3$-3-$C_6H_4$-CO- | fum. | 159-160 |
| 15 | 2 | 2 | $CH_3$-3-$C_6H_4$-$CH_2$- | difum. | 212-213 |
| 16 | 3 | 1 | $C_6H_5$-CO- | HCl | 226-227 |
| 17 | 3 | 1 | $C_6H_5$-$CH_2$- | difum. | 179-180 |
| 18 | 3 | 1 | Cl-3-$C_6H_4$-CO- | HCl | 182-185 |
| 19 | 3 | 1 | Cl-3-$C_6H_4$-$CH_2$- | difum. | 178-180 |
| 20 | 3 | 1 | $CH_3$-3-$C_6H_4$-CO- | HCl | 192-193 |
| 21 | 3 | 1 | $CH_3$-3-$C_6H_4$-$CH_2$- | difum. | 176-177 |
| 22 | 3 | 1 | $C_2H_5O$-3-$C_6H_4$-CO- | HCl | 170-173 |
| 23 | 3 | 1 | $C_2H_5O$-3-$C_6H_4$-$CH_2$- | diHCl* | 210-214 |

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type $5\text{-}HT_{1A}$. Les composés déplacent dans l'hippocampe du rat la liaison, sur ces récepteurs, d'un ligand spécifique marqué, la $[^3H]$-hydroxy-8 dipropylamino-2 tétraline (désignée ci-après par "$[^3H]$-8-OH-DPAT") décrite par Gozlan et coll., Nature, (1983), *305*, 140-142.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois pendant 10mn à 48000xg et en remettant le culot en suspension dans du tampon frais refroidi. Finalement on met le dernier culot en suspension dans le tampon pour arriver à une concentration de 100 mg de tissu de départ par ml de tampon à 50 mM. On laisse ensuite incuber à 37°C pendant 10 mn.

La liaison avec la $[^3H]$-8-OH-DPAT (1 nM) est déterminée par incubation de 100 µl de suspension de membranes dans un volume final de 1 ml de tampon contenant 10 µM de pargylline et 3 µM de paroxétine. Après une incubation de 5mn à 37° C on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la $[^3H]$-8-OH-DPAT est définie comme la quantité radioactive retenue sur les filtres et pouvant être inhibée par co-incubation dans la hydroxy-5 tryptamine à 10 µM. A une concentration de 1 nM de $[^3H]$-8-OH-DPAT la liaison spécifique représente de 70 à 80% de la radioactivité totale recupérée sur le filtre.

Pour chaque concentration de composés étudié on détermine le pourcentage d'inhibition de la liaison avec la $[^3H]$-8-OH-DPAT, puis la concentration $CI_{50}$, concentration qui inhibe 50% de la liaison.

Pour les composés de l'invention les $CI_{50}$ se situent entre 0,001 et 1 µM.

Un autre essai in vitro a, par ailleurs, montré que les composés selon l'invention ont une affinité vis-à-vis des récepteurs σ (sigma) des membranes du cortex cérébral du rat, Ce qui les désigne comme agents antipsychotiques potentiels.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977). On administre des doses cumulatives de composés à étudier (de 0,001 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électro-encéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DE_{50}$ se situent entre 0,003 et 3 mg/kg par la voie intraveineuse.

Les résultats des essais montrent que les composés de formule (I) possèdent, in vitro, une grande affinité et une sélectivité pour les récepteurs sérotoninergiques de type $5\text{-}HT_{1A}$, ainsi que pour les récepteurs de type sigma. In vivo ils montrent une activité agoniste ou agoniste partielle, au niveau de ces récepteurs.

Les composés de l'invention peuvent donc être utilisés pour le traitement des maladies et affections impliquant les récepteurs sérotoninergiques de type $5\text{-}HT_{1A}$ et/ou sigma de façon directe ou indirecte, notamment pour le traitement des états dépressifs, des états d'anxiété, des états psychotiques tels que la schizophrénie, des troubles du sommeil, et pour la régulation de la prise de nourriture, et également pour le traitement de désordres vasculaires, cardiovasculaires et cérébrovasculaires tels que l'hypertension ou la migraine.

A cet effet ils peuvent être présentés sous toutes formes appropriées à leur administration par voie orale ou parentérale, associés à tous excipients convenables, et dosés pour permettre une posologie journalière de 1 à 1000 mg.

**Revendications**

1. Composés de formule générale (I)

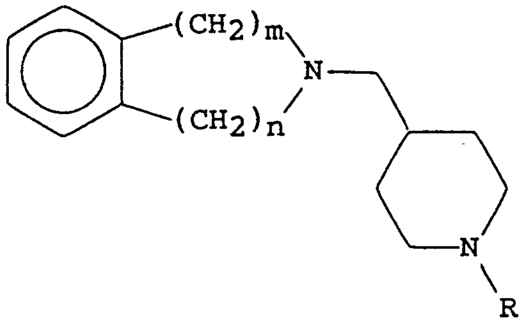

(I)

dans laquelle
soit chacun des symboles m et n représente le nombre 1,
soit chacun des symboles m et n représente le nombre 2,
soit m représente le nombre 3 et n représente le nombre 1, et
R représente soit un atome d'hydrogène, soit un groupe de formule générale -Z-R′ dans laquelle Z représente un groupe -CO- ou un groupe $-CH_2-$ et R′ représente un groupe phényle portant éventuellement de un à trois substituants choisis parmi les atomes d'halogènes et les groupes $(C_1-C_3)$alkyle linéaires ou ramifiés et $(C_1-C_3)$alcoxy linéaires ou ramifiés, ainsi que leurs sels d'addition à des acides.

2. Procédé de préparation de composés selon la revendication 1, caractérisé en ce qu'on fait réagir un composé de formule générale (II)

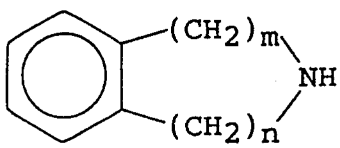

(II)

(dans laquelle m et n sont tels que définis dans la revendication 1) avec un tosylate de formule générale (III)

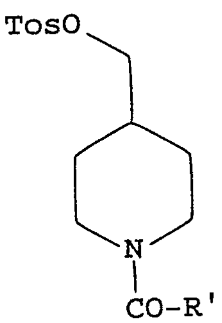

(III)

(dans laquelle Tos représente un groupe tosyle et R′ est tel que défini dans la revendication 1), soit en l'absence, soit en présence d'un solvant inerte tel que le diméthylformamide, le toluène ou le xylène, à une température de 20 à 150°C, et éventuellement en présence d'une base organique, telle qu'une amine tertiaire, ou minérale, telle qu'un carbonate ou hydrogénocarbonate alcalin,
obtenant ainsi un composé de formule générale (Ia)

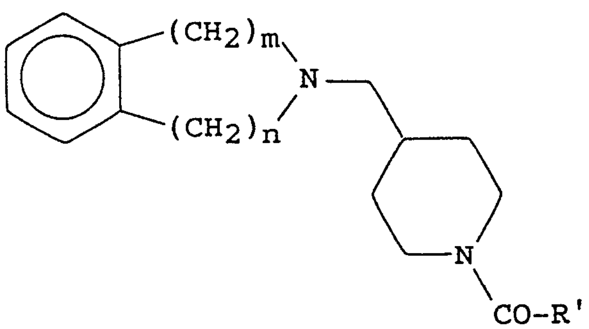

(Ia)

puis, si l'on désire préparer un composé de formule générale (I) où Z représente un groupe $-CH_2-$ on réduit ensuite le composé de formule générale (Ia) au moyen d'un hydrure simple ou complexe de bore ou d'aluminium, tel que l'hydrure de lithium et d'aluminium, l'hydrure d'aluminium, le complexe diborane/tétrahydrofuranne ou le complexe diborane/sulfure de méthyle, dans un solvant éthéré tel que l'éther diéthylique, le tétrahydrofuranne ou le dioxanne, à une température de 20 à 100°C.

3. Composition pharmaceutique caractérisée en ce qu'elle contient un composé selon la revendication 1 associé à un excipient pharmaceutique.

**Revendication pour les Etats contractants suivants : ES, GR**

Procédé de préparation de composés de formule générale (I)

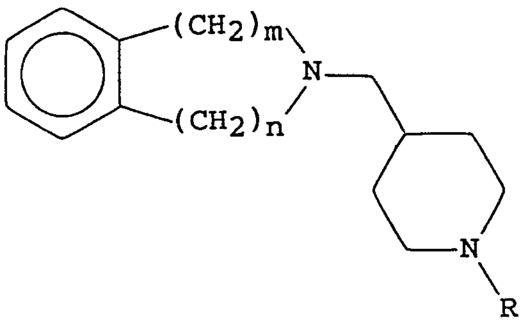

(I)

dans laquelle
soit chacun des symboles m et n représente le nombre 1,
soit chacun des symboles m et n représente le nombre 2,
soit m représente le nombre 3 et n représente le nombre 1, et
R représente soit un atome d'hydrogène, soit un groupe de formule générale -Z-R′ dans laquelle Z représente un groupe -CO- ou un groupe $-CH_2-$ et R′ représente un groupe phényle portant éventuellement de un à trois substituants choisis parmi les atomes d'halogènes et les groupes $(C_1-C_3)$alkyle linéaires ou ramifiés et $(C_1-C_3)$alcoxy linéaires ou ramifiés, procédé caractérisé en ce qu'on fait réagir un composé de formule générale (II)

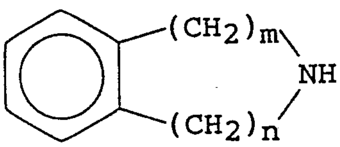

(II)

(dans laquelle m et n sont tels que définis ci-dessus) avec un tosylate de formule générale (III)

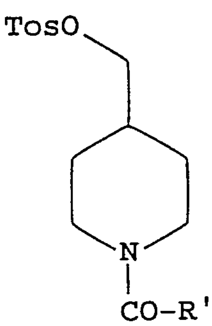

(III)

(dans laquelle Tos représente un groupe tosyle et R′ est tel que défini ci-dessus), soit en l'absence, soit en présence d'un solvant inerte tel que le diméthylformamide, le toluène ou le xylène, à une température de 20 à 150°C, et éventuellement en présence d'une base organique, telle qu'une amine tertiaire, ou minérale, telle qu'un carbonate ou hydrogénocarbonate alcalin,
obtenant ainsi un composé de formule générale (Ia)

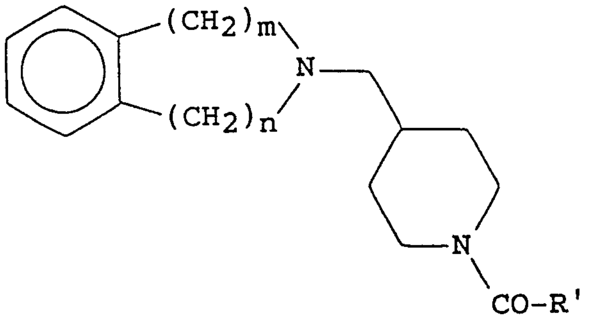

(Ia)

puis, si l'on désire préparer un composé de formule générale (I) où Z représente un groupe $-CH_2-$ on réduit ensuite le composé de formule générale (Ia) au moyen d'un hydrure simple ou complexe de bore ou d'aluminium, tel que l'hydrure de lithium et d'aluminium, l'hydrure d'aluminium, le complexe diborane/tétrahydrofuranne ou le complexe diborane/sulfure de méthyle, dans un solvant éthéré tel que l'éther diéthylique, le tétrahydrofuranne ou le dioxanne, à une température de 20 à 100° C.

Office européen des brevets

# RAPPORT DE RECHERCHE EUROPEENNE

Numero de la demande

EP 89 40 1899

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A-2 177 692 (BRISTOL-MYERS)<br>* Revendications 1,38 *<br>----- | 1,3 | C 07 D 401/06<br>A 61 K 31/445<br>A 61 K 31/55 |
| | | | **DOMAINES TECHNIQUES RECHERCHES (Int. Cl.5)** |
| | | | C 07 D 401/00<br>A 61 K 31/00 |

Le présent rapport a été établi pour toutes les revendications

| Lieu de la recherche | Date d'achèvement de la recherche | Examinateur |
|---|---|---|
| LA HAYE | 26-09-1989 | CASADO Y MARTIN DE MERCA |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)